Europäisches Patentamt

European Patent Office

Office européen des brevets

(19)

(11) Publication number: **0 219 969 B1**

# EUROPEAN PATENT SPECIFICATION

(12)

(45) Date of publication of patent specification: **22.01.92**  (51) Int. Cl.⁵: **A61F 13/15**

(21) Application number: **86306995.1**

(22) Date of filing: **10.09.86**

(54) **Diaper provided with an improved elastic fitting.**

(30) Priority: **13.09.85 FR 8513641**

(43) Date of publication of application:
**29.04.87 Bulletin 87/18**

(45) Publication of the grant of the patent:
**22.01.92 Bulletin 92/04**

(84) Designated Contracting States:
**BE CH DE GB IT LI NL**

(56) References cited:
**FR-A- 2 289 131**
**FR-A- 2 388 515**
**FR-A- 2 421 571**
**GB-A- 2 085 281**
**GB-A- 2 130 888**

(73) Proprietor: **PTM, Inc.**
**Corporation Trust Center 1209 Orange Street**
**Wilmington Delaware 19801(US)**

(72) Inventor: **Daugan, Jean-Claude**
**5 Residence des 3 Forets**
**F-78380 Bougival(FR)**
Inventor: **LeRoy, Francis**
**3 Rue de la Pente**
**Jouy le Moutier F-95000 Cergy(FR)**

(74) Representative: **Leszczynski, André et al**
**CABINET NONY & CIE. 29 rue Cambacérès**
**F-75008 Paris(FR)**

EP 0 219 969 B1

## Description

The present invention relates to diapers (or napkins), particularly discardable diapers, comprising a flexible and impermeable support sheet, a flexible and permeable upper sheet joined to the support sheet over at least a portion of the periphery of the diaper. A pad of absorbent material is placed between the upper sheet and the support sheet and elastic means positioned over at least a portion of the periphery of the diaper designed to be applied elastically onto the body of a user of this diaper.

Numerous diapers are now found provided with elastic members at the level of the crotch designed to form a barrier around the thighs of the user of the diaper. Such diapers have different shapes; thus, they may be rectangular with longitudinal edges, as the case may require, folded back to reduce the width at the crotch or have a cut-out at the level of this crotch in order to reduce the width of the diaper and thus to constitute a so-called anatomical shape.

These diapers provided with elastic elements at the crotch are generally well adapted to the body of the user and do not show leakages around the thighs at least when they are not saturated with liquid or poorly positioned.

However, leakages frequently occur around the waists of babies according to the position of the latter. Such leakages are associated with the morphology of the baby, who does not have a muscular abdominal belt. In spite of all the precautions which can be taken on the placing in position of the napkin, the latter, which is capable of being distorted at the level of the waist, begins, after a certain number of changes in position of the baby, to gape at the level of the waist of the latter which thus results in risk of leakages in the following cases:

- Direct leakages especially in reclining position. The flow rate of the urination, generally being very much greater at the level at which the urine flows through the permeable upper sheet, the urinary liquid escapes at the spot where the complete change is not tight. This type of leakage can also occur on occasional pressure exerted on the absorbent material of the pad when the latter is close to saturation;
- Direct leakages by directional orientation of the urination stream towards the place at the waist of the infant where the diaper has a tendency to gape, which is the case with boys;
- Leakage by capillary pumping when an undergarment of hydrophilic material slips between the skin and the diaper which gapes at the level of the waist.

To avoid such leakages and to apply the corresponding portion of the diaper better to the waist of the user, certain diapers have been provided with elastic members in the form of strips, tapes or threads fixed to at least a part of the width of the diaper and at the border of the latter between the support sheet and the top sheet. Such diapers are described in patents FR-A-82 04 390 and 84 08 289.

However, these improvements associated with a more hermetic application of the diaper to the user do not make any contribution to provide means intended to place the inner space of the diaper in communication with the outside and in this manner to permit the passage of gaseous fluids and, thus, to ventilate the inside of the diaper to contribute to the comfort of the user.

In the prior art there are found diapers which, to arrange means of communication of their inner space with the outside when the diaper is worn, provide perforations or microperforations formed on the support sheet which normally is completely impermeable. It appears, however, that in the case of microperforations, the flow rate of gaseous fluid that the diaper can exchange with the outside is relatively low. In the case of perforations, the fluid tightness of the diaper with respect to liquid leakages is compromised, the permeability of the support sheet being too degraded.

The diaper according to the invention is of a type comprising a flexible and impermeable support sheet a flexible and permeable top sheet joined to the support sheet over at least a portion of the periphery of the diaper, a pad of absorbent material arranged between the top sheet and the support sheet and at least one strip of an elastic material, positioned over at least a portion of the periphery of the diaper, designed to be applied elastically to the body of a user of this diaper, wherein said at least one strip is fixed on one surface to the support sheet and, on the opposite surface, to the top sheet to connect these sheets together, and is arranged between the support sheet and the top sheet so that the inner space of the diaper communicates with the outside of the latter through the thickness of each of said strips, the outer and inner longitudinal edges of said at least one strip communicating respectively with the outside of the diaper and the inner space of the latter.

Diapers of this type are disclosed in GB-A-2 130 888. In this document the strips of elastic material define open channels extending between the interior of the diaper and the margin thereof thus allowing the exchange of air and vapour between the interior of the diaper and the surrounding atmosphere. However such channels are note suitable to prevent liquid leakages.

GB-A-2 085 281 discloses a diaper including at least one strip of an elastic material of open cell foam arranged between a support sheet and a top sheet. The inner surfaces of the support sheet and the top sheet are bonded to each other by an adhesive along their outer edges so that the inner space of the diaper including the absorbent pad does not communicate with the atmosphere. Furthermore in this document the strips of elastic material are made of a water absorptive material which absorbs excrement liquid and thus is not suitable to prevent liquid incoming at its inner edge from flowing outside.

Accordingly the above mentioned adhesive bonding is necessary to avoid liquid leakages.

The object of the invention is to provide a diaper which permits good elastic application especial by around the waist of the user whilst providing effective means for placing the inner volume of the diaper in communication with the outside whilst preventing liquid leakages.

The diaper according to the invention is characterized in that the at least one strip is made of an open cell water-repellent foam material, wherein the foam material of said at least one strip and the cross-section of the latter are such that the strip imposes a pressure drop on the flow of a liquid through the strip whilst being permeable to gaseous fluids.

Other features of the invention are disclosed in the subclaims.

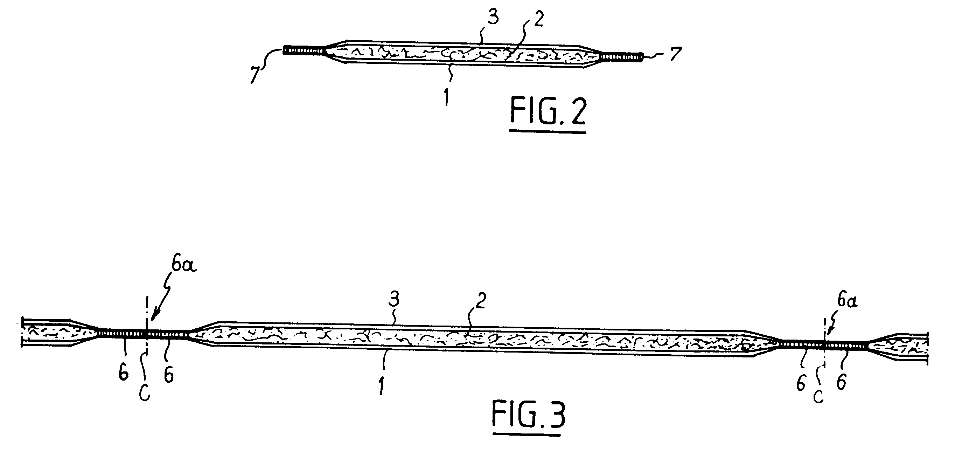

Fig. 3 is a view in longitudinal section of a napkin in which are formed several diapers which extend longitudinally whilst being each joined through their transverse edges to the corresponding neighboring diapers.

The diaper illustrated in the figures is intended to be thrown away after use and has a profiled shape by arranging a portion of reduced width for positioning at the level of the crotch of the user. This diaper comprises principally a flexible and impermeable support sheet 1, for example, of polyethylene, a pad 2 of absorbent material, for example, of defibered wood pulp, of cellulose wadding or any other absorbent material, such as so-called super-absorbent polymers, and a flexible and permeable top sheet, in particular permeable to urine, formed of, for example, a non-woven cloth or of perforated plastic film.

The absorbent pad 2 is placed between the support sheet 1 and top sheet 3, the latter being joined together over the whole of their periphery, for example, by gluing or heat-sealing, to thus enclose the pad within an internal space of the diaper bounded between the sheets.

In addition, to ensure the positioning of the diaper on the user, a fastening system is provided at one end of each longitudinal edge of the latter in the form of an adhesive tongue 5, each designed to cooperate with the support sheet 1 on a corresponding side of the opposite end of the diaper on its placing in position on the user.

The diaper comprises in addition a first elastic system formed by two elastic strips 6, each arranged along an intermediate portion of a corresponding transverse edge of the diaper and a second elastic system formed from two elastic strips 7, each extending longitudinally along a corresponding longitudinal edge of the diaper at the level of the crotch.

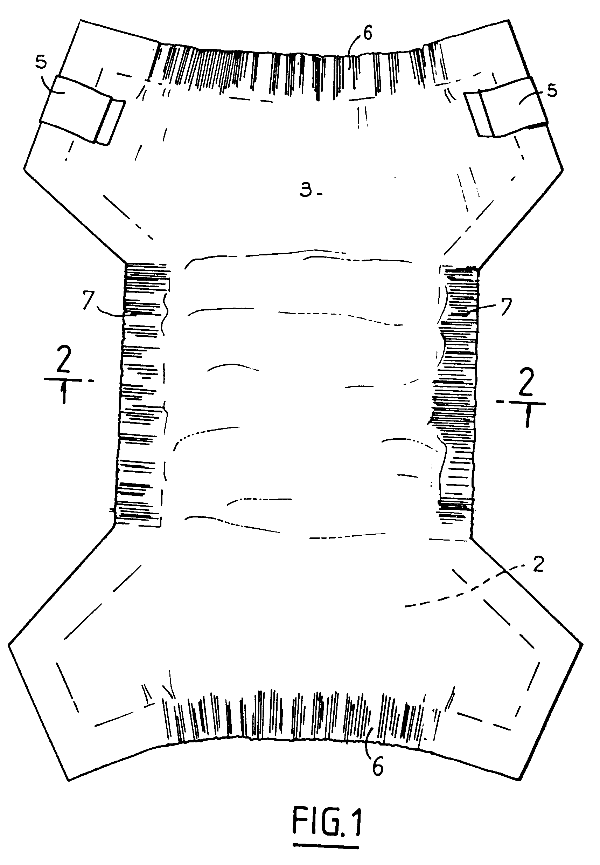

The strips 6, 7 of the first and second elastic systems are each fixed and tensioned between the support sheet 1 and the top sheet 3, thus forming gathered sections visible in Fig. 1.

These strips 6, 7 extend along the width over the whole of the interval comprised between the neighboring edge of the absorbent pad 2 and the corresponding edge of the diaper itself. Thus, at the level of the peripheral portions of the diaper where these strips 7 extend, the upper and support sheets are joined together through strips which are each fixed, for example, by gluing or by thermowelding, on one of their surfaces to the support sheet 1 and, on their opposite surface, to the top sheet 3. This gluing of the strips to the sheets must be limited to the surfaces of these strips without extending into the thickness of the latter in order to avoid plugging the cells of the foam which must remain open.

The adhesion of the support sheet 1 and top sheet 3 to each strip 6 or 7 must, preferably, be such that no flow path can exist for a fluid between each of these sheets and the corresponding surface of the strips so as to avoid liquid leakages.

It will be noted, in addition, in Figs. 2 and 3, that the outer longitudinal edge of each strip 6 or 7 and the neighboring edges of the top sheet 3 and support sheet 1 are contained within almost the same plane to leave thus visible the outer longitudinal edge of the strip, the inner longitudinal edge of the latter communicating between the sheets with the inner space of the diaper wherein the pad 2 extends. This arrangement is, in fact, obtained by cutting simultaneously the two sheets and the strips at the time of fabrication of the diaper. It is seen by referring to Fig. 3 that each strip 6 comes from a corresponding strip 6a cut out longitudinally approximately at its middle to form two strips 6, each belonging to two distinct diapers.

In Fig. 3, the diapers not yet detached from one another are assembled in one layer where they follow one another by being joined at their transverse edges, a strip 6a extending transversely between each of these unseparated diapers by being fixed between the support sheet 1 and top sheet 3 still in the form of continuous films. From this layered structure, the diapers are then detached successively from one another by transverse cutting off along the lines C (Fig. 3) approximately at the middle of each strip 6a which has therefore a width substantially double that of each strip 6. The outer and inner longitudinal edges of the strip 6 or 7 communicate respectively with the outside of the diaper and the inner space of the latter as will be demonstrated below in the rest of the description.

In accordance with the invention, each strip 6, 7 is formed of an open cell foam elastic material, for example, of polyester, of polyether (polyurethane) or any other suitable material.

By way of example, it is possible to use polyurethane foam of density equal to about 34 kg/m³.

The role of these strips is not only to confer on the places of the diaper where they are placed, a certain elasticity intended to apply elastically and as hermetically as possible, the diaper to the corresponding parts of the body of the user, but also to permit the placing in communication of the inner space of the diaper with the outside of the latter when the diaper is worn by the user, this communication taking place through the thickness of each strip through their open cells which permits advantageous ventilation of this inner space.

The thickness of the strips 6, 7 may be comprised between about 1 and 5mm, preferably between about 2 and 3mm, the thickness of the strips 6 extending transversely, and preferably less than about 50mm, the width of the strips 7 extending longitudinally being less than about 35mm and, preferably, equal to about 25mm.

The elasticity of the foam strips 6 must be sufficient to cover the maximum variations in waist size of the user (10cm for an infant of about 10 to 12kg). In addition, the thickness of the strips 6 or 7 must be sufficient to ensure good strength but must not be too great so as to limit the force necessary for elongation of each of the strips in order to preserve thus the comfort of the user and to permit the closing of the open cells by longitudinal stretching or compression in the thickness on tension of the strip or compression of the latter in order to avoid forced leakages of liquid through the foam.

The porosity of the foam material and the transverse section of the strips are such that the latter induce a high pressure drop with respect to the flow of the liquid through each strip which remains permeable to gaseous fluids. This selective character of the permeability with regard to gases rather than with regard to liquids is, in addition, increased by the hydrophobic nature of the foam material which thus counters the flow of aqueous liquids upon urination.

Each strip thus ensures the fluid tightness of the application of the diaper to the body of the user whilst simultaneously permitting ventilation of the inner space of this diaper.

In use, this diaper is placed on the user so that the strips 7 situated at the level of the crotch grip the thighs of this user and so that the strips 6 are applied to the waist of the latter.

The longitudinal elasticity of the foam strips permits the waist of the infant to be gripped by soft contact of the diaper around it in any position, eliminating direct leakages, a comfortable grip being also reproduced around the thighs. The elastic foam through its composition permits a notable elongation even for a width and thickness of the strip which are fairly large under the action of a weaker force with respect to other conventionally used elastic materials.

The thickness of the foam permits, moreover, a second elastic effect to be contributed by compression of the cells open in the direction of the thickness. This property improves the comfort of the user whilst eliminating the risk of leakages by pressure on the absorbent material, the cross-section of each strip being in fact such that, under the effect of a sufficient force of compression or of traction, the open cells are closed by compression or stretching of the strip respectively.

The elastic properties in extension and in compression of the strips thus permit an anti-leakage barrier to be obtained which is as effective as that which it could be hoped to obtain by means of a compact elastic material whilst showing better flexibility of application to the body of the user, which procures for the latter an important sensation of comfort.

According to yet another advantage, the use of open cell foam for the elastic strips enables the ventilation of the inner space of the diaper whilst resisting passages of fluids.

Although the example of a diaper previously described to illustrate the invention comprises an elastic system intended to grip the waist of the user and an elastic system intended to grip the thighs of the latter, it is clear that one or other of these elastic systems may be used independently of the other.

However, the preferred embodiment of the invention recommends placing the open cell foam strips previously described at the level of the transverse edges of the diaper to the extent that it is this spot, intended to be applied to the waist of the user, that the foam strips will play most effectively their role of means of communication of the inner space of the diaper with the outside. In fact, the open cells of the foam strips provided on the longitudinal edges of the diaper have, when the latter is worn by a user, a tendency to be held closed when the crotch of the diaper is applied hermetically around the thighs and the corresponding strips are then stretched. The thighs are only subjected to a slight variation in circumference as a function of the different positions of the user, the state of initial tension of the foam strips provided the level of the crotch is hence always more or less substantially preserved and, for this reason, the cells have less tendency to be kept open.

On the other hand, when these foam strips are placed on the transverse edges of the diaper, they become applied to the waist of the user and it is well known that, at the level of the abdomen, the body varies notably in circumference as a function of the respiratory movements or abdominal muscular contractions. These variations in circumference permit, even if the diaper has been applied in a stretched manner around the waist, the corresponding foam strips to be substantially relaxed and thus permit at least periodically the opening of the cells if the latter are otherwise closed and favor the ventilation of the inner space of the diaper.

The diaper may be of any conventionally known general shape, other than those previously described, for example, rectangular, in the form of an H or with any other anatomical cutout. According to another modification, a space may be formed between one elastic strip and the neighboring edge of the absorbent pad if the inner longitudinal edge of the strip can communicate with the inner space of the diaper. According to another modification, the outer edge of one elastic foam strip may be stretched more at the inside or more at the outside of the neighboring edges of the top and support sheets with respect to the diaper, the essential thing being that the outer longitudinal edge of this strip should communicate with the outside of the diaper.

It is well understood that the invention is not limited to use for babies, but can also be used by a user of any age, for example, an incontinent adult, the dimensions of the diaper being then adapted to the corpulence of the user.

## Claims

1. Diaper comprising a flexible and impermeable support sheet (1), a flexible and permeable top (3) sheet joined to the support sheet over at least a portion of the periphery of the diaper, a pad (2) of absorbent material arranged between the top sheet and the support sheet and at least one strip (6, 7) of an elastic material, positioned over at least a portion of the periphery of the diaper, designed to be applied elastically to the body of a user of this diaper, wherein said at least one strip (6,7) is fixed on one surface to the support sheet (1) and, on the opposite surface, to the top sheet (3) to connect these sheets together, and is arranged between the support sheet and the top sheet so that the inner space of the diaper communicates with the outside of the latter through the thickness of each of said strips, the outer and inner longitudinal edges of said at least one strip communicating respectively with the outside of the diaper and the inner space of the latter, characterized in that said at least one strip (6,7) is made of an open cell water-repellent foam material, wherein the foam material of said at least one strip and the cross-section of the latter, are such that the strip imposes a pressure drop on the flow of a liquid through the strip whilst being permeable to gaseous fluids.

2. Diaper according to claim 1, wherein the cross-section of said at least one strip is such that for a sufficient traction or compression force exerted on the strip, the open cells close so as to avoid any flow of fluid.

3. Diaper according to any one of the preceding claims, wherein said at least one strip extends along its width over the whole interval comprised between the neighbouring edge of the pad and the corresponding edge of the diaper.

4. Diaper according to any one of the preceding claims, wherein it comprises two strips (6) each extending transversely over at least a portion of the width of the diaper in the vicinity of a corresponding transverse edge of the latter.

5. Diaper according to any one of the preceding claims, comprising two strips (7) each extending longitudinally over at least a portion of the length of the diaper in the vicinity of a corresponding longitudinal edge of the latter.

## Revendications

1. Couche comportant une feuille support flexible et imperméable (1), une feuille supérieure flexible et perméable (3) réunie à la feuille support sur au moins une partie de la périphérie de la couche, un tampon (2) en matériau absorbant disposé entre la feuille supérieure et la feuille support et au moins une bande (6,7) en un matériau élastique, positionnée sur au moins une partie de la périphérie de la couche, conçue pour être appliquée élastiquement au corps d'un utilisateur de cette couche, dans laquelle ladite au moins une bande (6,7) est fixée sur une surface de la feuille support (1) et, sur la face opposée, à la feuille supérieure (3) pour réunir ces feuilles l'une à l'autre, et est disposée entre la feuille support et la feuille supérieure de sorte que l'espace intérieur de la couche communique avec l'extérieur de celle-ci par l'intermédiaire de l'épaisseur de chacune desdites bandes, les bords longitudinaux extérieurs et intérieurs de ladite au moins une bande communiquant respectivement avec l'extérieur de la couche et l'espace intérieur de celle-ci, caractérisée en ce que ladite au moins une bande (6,7) est constituée d'un matériau en mousse à cellules ouvertes,hydrophobe, dans laquelle le matériau en mousse de ladite au moins une bande et la section transversale de cette dernière, sont tels que la bande provoque une chute de pression sur l'écoulement d'un liquide à travers la bande tout en étant perméable à des fluides gazeux.

2. Couche selon la revendication 1, dans laquelle la section transversale de ladite au moins une bande est telle que pour une traction suffisante ou une force de compression exercée sur la bande, les cellules ouvertes se ferment de manière à éviter tout écoulement de fluide.

3. Couche selon l'une quelconque des revendications précédentes, dans laquelle ladite au moins une bande s'étend le long de sa largeur sur tout l'intervalle compris entre le bord adjacent du tampon et le bord correspondant de la couche.

4. Couche selon l'une quelconque des revendications précédentes, qui comporte deux bandes (6) s'étendant chacune transversalement sur au moins une partie de la largeur de la couche à proximité d'un bord transversal correspondant de cette dernière.

5. Couche selon l'une quelconque des revendications précédentes, comportant deux bandes (7) s'étendant chacune longitudinalement sur au moins une partie de la longueur de la couche à proximité d'un bord longitudinal correspondant de celle-ci.

## Patentansprüche

1. Windel, umfassend eine flexible und undurchlässige Trägerlage (1), eine flexible und durchlässige obere Lage (3), die mit der Trägerlage wenigstens im Bereich eines Teils des Außenumfangs der Windel verbunden ist, ein Polster (2) aus absorbierendem Material, das zwischen der oberen Lage und der Trägerlage angeordnet ist, und wenigstens einen Streifen (6, 7) aus einem elastischen Material, der wenigstens im Bereich eines Teils des Außenumfangs der Windel angeordnet und dafür vorgesehen ist, elastisch am Körper eines Benutzers dieser Windel befestigt zu werden, worin der wenigstens eine Streifen (6, 7) auf einer Oberfläche an der Trägerlage (1) und an der gegenüberliegenden Oberfläche an der oberen Lage (3) befestigt ist, um diese beiden Lagen miteinander zu verbinden, und zwischen der Trägerlage und der oberen Lage so angeordnet ist, daß der Innenbereich der Windel mit der Außenseite der Windel durch die Dicke jedes der Streifen in Verbindung steht, wobei die Außen- und Innen-Längskanten des wenigstens einen Streifens jeweils mit der Außenseite der Windel und dem Innenbereich der Windel in Verbindung stehen, **dadurch gekennzeichnet,** daß der wenigstens eine Streifen (6, 7) aus einem offenzelligen, wasserabstoßenden Schaummaterial hergestellt ist, worin das Schaummaterial des wenigstens einen Streifens und der Querschnitt des Streifens derart sind, daß der Streifen einen Druckabfall auf den Fluß einer Flüssigkeit durch den Streifen ausübt, während er für gasförmige Fluide durchlässig ist.

2. Windel nach Anspruch 1, worin der Querschnitt des wenigstens einen Streifens so ist, daß sich bei Ausübung einer ausreichenden Zugkraft oder Kompressionskraft auf den Streifen die offenen Zellen schließen, um einen Fluid-Fluß zu vermeiden.

3. Windel nach irgendeinem der vorangehenden Ansprüche, worin sich der wenigstens eine Streifen in seiner Breite über den gesamten Abstand zwischen der benachbarten Kante des Polsters und der entsprechenden Kante der Windel erstreckt.

4. Windel nach irgendeinem der vorangehenden Ansprüche, welche zwei Streifen (6) umfaßt, von denen sich jeder quer über wenigstens einen Teil der Breite der Windel in der Nähe einer entsprechenden Quer-Kante der Windel erstreckt.

5. Windel nach irgendeinem der vorangehenden Ansprüche, welche zwei Streifen (7) umfaßt, von denen sich jeder längs über wenigstens einen Teil der Länge der Windel in der Nähe einer entsprechenden Längskante der Windel erstreckt.

FIG.1

FIG.2

FIG.3